# EUROPEAN PATENT SPECIFICATION

(11) **EP 4 382 034 B1**
(45) Date of publication and mention of the grant of the patent: **18.03.2026**
(21) Application number: 22211424.1
(22) Date of filing: 05.12.2022
(51) Int. Cl.: A61B 3/14

(54) **MIRROR ALIGNMENT MECHANISM FOR OPHTHALMIC IMAGING SYSTEMS**
SPIEGELAUSRICHTUNGSMECHANISMUS FÜR OPHTHALMISCHE BILDGEBUNGSSYSTEME
MÉCANISME D'ALIGNEMENT DE MIROIR POUR SYSTÈMES D'IMAGERIE OPHTALMIQUE

(43) Date of publication of application: 12.06.2024
(73) Proprietor: Optos plc, Dunfermline, Scotland KY11 8GR (GB)
(72) Inventor: Hollman, Stephen, Dunfermline, Scotland, KY11 8GR (GB)
(74) Representative: Hoffmann Eitle

(56) References cited:
- JP-A- H08 308 798
- US-B2- 6 830 335

## Description

### [Field]

Example aspects herein generally relate to the field of ophthalmic imaging systems and, in particular, to optical assemblies for coupling light sources and photodetectors to imaging optics in ophthalmic imaging systems.

### [Background]

Ophthalmic imaging systems typically include a light source arranged to generate a light beam, and a photodetector, as well as an optical system which is arranged to illuminate a portion of an eye (e.g. the retina) with light from the light beam and collect light from the illuminated portion of the eye. An optical assembly for coupling the light source and the photodetector to the optical system of an ophthalmic imaging apparatus, which is also referred to as a "turret module" herein, is provided to guide the light beam from the light source to the optical system, and to convey the light collected by the optical system towards the photodetector. Conventional turret modules have a housing, through which the collected ("return") light passes along an optical path towards the photodetector, and through which the light beam from the light source propagates towards the optical system in the opposite direction along the optical path. In some conventional ophthalmic imaging systems, such as the Optos^{™} California^{™} system, the light source is arranged to emit the light beam through an opening in the turret module, with the emitted light beam being at about 90 degrees to the optical path. Within the turret module, the light beam is coupled into the optical path by a fixed dot mirror (i.e. a small mirror formed on a glass substrate), which is orientated at about 45 degrees to the optical path.

However, the emission directions of light beams emitted from typical light sources tend to deviate from a nominal emission direction, owing to manufacturing tolerances, and the light sources rarely have means for adjusting the light beam direction. The light beam entering the turret module is therefore usually not aligned with the dot mirror precisely enough for the reflected light beam to propagate along the optical axis. Attempts to compensate for this misalignment are usually made downstream of the turret module, by adjusting one or more components in the optical system. Even when such adjustments, which are normally made during production of the ophthalmic imaging system, successfully compensate for the misalignment, there remains the problem that factors such as movement of light source components during shipping of the ophthalmic imaging system, and/or ageing of the components in the longer term, for example, may cause a subsequent misalignment of the light beam with the optics of the imaging system and a consequent deterioration in its performance.

Further background is provided in US 6,830,335 B2, which discloses a binocular indirect laser ophthalmoscope is provided comprising binocular eyepieces each having an optical axis defining an optical plane, the ophthalmoscope having a central viewing axis lying in the optical plane; and an optical element adapted to position a laser beam into the optical plan substantially on the viewing axis and substantially parallel to the viewing axis.

### [Summary]

There is provided, in accordance with a first example aspect herein, an ophthalmic imaging apparatus comprising: a light source arranged to generate a light beam, a photodetector, and an optical system arranged to illuminate a portion of an eye with light from the light beam and collect light from the illuminated portion of the eye. The ophthalmic imaging apparatus further comprises an optical assembly, which is arranged to guide the light beam from the light source to the optical system, and to convey the light collected by the optical system towards the photodetector. The optical assembly comprises a housing through which the light beam from the light source propagates towards the optical system in a first direction along an optical path during use of the ophthalmic imaging apparatus, and through which the light collected by the optical system propagates towards the photodetector in a second direction along the optical path during use of the ophthalmic imaging apparatus, wherein the second direction is opposite to the first direction. The housing comprises an opening for allowing the light beam generated by the light source to enter the housing. The optical assembly further comprises an arm which extends into the housing, and a reflective element mounted on the arm, the reflective element comprising a reflective surface for reflecting the light beam passing through the opening during use of the ophthalmic imaging apparatus. The optical assembly further comprises an adjustable attachment mechanism, which is arranged to attach the arm to the housing and to allow the reflective element to be adjusted by at least one of: rotating the reflective element about a first axis of rotation passing through a point on the reflective surface, the first axis of rotation being perpendicular to the optical path; rotating the reflective element about a second axis of rotation passing through the point on the reflective surface, the second axis of rotation being perpendicular to the first axis of rotation and the optical path; or translating the reflective element along the optical path, such that, during use of the ophthalmic imaging apparatus, the reflective element is arranged to reflect the light beam passing through the opening to propagate along the optical path in the first direction.

Where the adjustable attachment mechanism is arranged to allow the reflective element mounted on the arm to be adjusted by rotating the reflective element about the first axis of rotation, the adjustable attachment mechanism may comprise a first contact surface which faces the reflective element, wherein the first contact surface has a shape of a part of a surface of revolution about the first axis of rotation. Furthermore, the arm may comprise comprises one or more second contact surfaces arranged to contact the first contact surface at points that are equidistant from the first axis of rotation and have different respective angular positions about the first axis of rotation, wherein the one or more second contact surfaces may be slidable over the first contact surface such that the reflective element is rotatable about the first axis of rotation.

Alternatively, where the adjustable attachment mechanism is arranged to allow the reflective element mounted on the arm to be adjusted by rotating the reflective element about the first axis of rotation, the arm may comprise a first contact surface which faces away from the reflective element, wherein the first contact surface has a shape of a part of a surface of revolution about the first axis of rotation, the adjustable attachment mechanism may comprise one or more second contact surfaces arranged to contact the first contact surface at points that are equidistant from the first axis of rotation and have different respective angular positions about the first axis of rotation, and the one or more second contact surfaces are slidable over the first contact surface such that the reflective element is rotatable about the first axis of rotation.

In addition, the adjustable attachment mechanism may further comprise: a first moveable member attached to the adjustable attachment mechanism and having an end abutting a side of the arm, wherein the first moveable member is moveable relative to the adjustable attachment mechanism to vary a position of the end of the first moveable member relative to the adjustable attachment mechanism; and a first resilient member arranged to force the side of the arm against the end of the first moveable member. The first moveable member, when moved relative to the adjustable attachment mechanism, may be arranged to cause the arm to move such that the one or more second contact surfaces slide over the first contact surface.

In addition, the first moveable member attached to the adjustable attachment mechanism may comprise a first screw which has been screwed through a first threaded portion of the adjustable attachment mechanism. An end of the first screw may abut the side of the arm, and the first screw, when rotated to move through the first threaded portion, may cause the arm to move such that the one or more second contact surfaces slide over the first contact surface.

Where the adjustable attachment mechanism additionally or alternatively allows the reflective element mounted on the arm to be adjusted by rotating the reflective element about the second axis of rotation, the adjustable attachment mechanism may further comprise a supporting portion attached to the arm, and the supporting portion may be rotatable about a pivot which is aligned with the second axis of rotation such that the reflective element mounted on the arm is rotatable about the second axis of rotation.

In this case, the adjustable attachment mechanism may comprise a second moveable member attached to the adjustable attachment mechanism and having an end abutting a side of the supporting portion, and a second resilient member arranged to force the side of the supporting portion against the end of the second moveable member, wherein the second moveable member, when moved relative to the adjustable attachment mechanism, is arranged to cause the supporting portion to rotate about the pivot, thereby causing the reflective element to rotate about the second axis of rotation. The supporting portion may comprise the first contact surface.

The second moveable member attached to the adjustable attachment mechanism may comprise a second screw which has been screwed through a second threaded portion of the adjustable attachment mechanism, wherein an end of the second screw abuts the side of the supporting portion, and wherein the second screw, when rotated to move through the second threaded portion, causes the supporting portion to rotate about the pivot. Additionally or alternatively, the arm may be biased against the supporting portion by a first attachment screw which is disposed in a threaded portion of the supporting portion, and a first attachment resilient member which is held in compression between a head of the first attachment screw and a surface of the arm.

Where the adjustable attachment mechanism additionally or alternatively allows the reflective element mounted on the arm to be adjusted by translating the reflective element along the optical path, the adjustable attachment mechanism may comprise a base portion which is slidably attached to the housing so as to be slidable along the housing, in a direction parallel to the optical path, such that the reflective element mounted on the arm is translatable along the optical path. In this case, the optical assembly may further comprise a third moveable member attached to the housing and having an end abutting a side of the base portion, and a third resilient member arranged to force the side of the base portion against the end of the third moveable member, wherein the third moveable member, when moved relative to the housing, is arranged to cause the base portion to slide along the housing, in the direction parallel to the optical path, thereby causing the reflective element mounted on the arm to be translated along the optical path. The third moveable member attached to the housing may comprise a third screw, which has been screwed through a third threaded portion of the adjustable attachment mechanism, wherein an end of the third screw abuts the side of the base portion, and wherein the third screw, when rotated to move through the third threaded portion, causes the base portion to slide along the housing, in the direction parallel to the optical path.

In example embodiments wherein the adjustable attachment mechanism comprises a supporting portion and a base portion, as set out above, the supporting portion may be attached to the base portion, and the pivot may be provided on the base portion and aligned with the second axis of rotation such that the reflective element mounted on the arm is rotatable about the second axis of rotation. In this case, the supporting portion may be attached to the base portion by a second attachment screw which is disposed in a threaded portion of the base portion, and a second attachment resilient member which is held in compression between a head of the second attachment screw and a surface of the supporting portion.

The base portion may be slidably attached to the housing by a third attachment screw which is disposed in a threaded portion of the housing, and a third attachment resilient member, which is held in compression between a head of the third attachment screw and a surface of the base portion.

In example embodiments wherein the adjustable attachment mechanism comprises the first threaded portion, the second threaded portion, and the third threaded portion, these threaded portions may be arranged to extend along a direction parallel to the optical path, such that the first screw, the second screw and the third screw can each be rotated about respective axes that are parallel to one another.

In any of the optical assemblies set out above, a projection of the arm on a plane perpendicular to the optical path may have an area which is less than 10 % (more preferably, less than 5 %) of an area of a cross-section of the housing or of the light collected by the optical system that propagates through the housing during use of the ophthalmic imaging apparatus.

The light source may be slidably attached to the ophthalmic imaging apparatus such that a location on the reflective element, at which the light beam generated by the light source is incident on the reflective element, is adjustable along an axis perpendicular to the optical path.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an ophthalmic imaging apparatus 100 comprising an optical assembly 150 according to an example embodiment herein.
Figure 2 is a schematic illustration of an example of a scanning system 130 forming part of the ophthalmic imaging apparatus 100.
Figure 3A is an isometric view of an optical assembly 200 of the example embodiment mounted onto a frame 160 of an ophthalmic imaging apparatus.
Figure 3B is an isometric view of a casing 114 of the light source 110 of the ophthalmic imaging apparatus 100.
Figure 4A is a first isometric view of an optical assembly 200 of the example embodiment.
Figure 4B is a second isometric view of the optical assembly 200 of the example embodiment.
Figure 4C is a cross-sectional view of the optical assembly 200 of the example embodiment, taken in a plane parallel to the x-z plane that includes the optical path.
Figure 5A is a cut-away view illustration of a part of the optical assembly 200 of the example embodiment, which includes a housing 210, an arm 220, a prism mirror 230, and portions of an adjustable attachment mechanism including a supporting portion 240 and a base portion 245.
Figure 5B is a view of the housing 210 of the of the optical assembly 200.
Figure 5C is a view of the arm 220 of the of the optical assembly 200.
Figure 5D is a view of the supporting portion 240 of the of the optical assembly 200.
Figure 5E is a view of the base portion 245 of the of the optical assembly 200.
Figure 6 is a side view of some components of the optical assembly 200 of the example embodiment, including the supporting portion 240 of the adjustable attachment mechanism, the arm 220, a first resilient member 303 and a first adjustable member 250.
Figure 7A is a side view of some components of the optical assembly 200 of the example embodiment, including the supporting portion 240 and base portion 245 of the adjustable attachment mechanism, the arm 220, a pivot 401, a second adjustable member 251 and a second resilient member 402.
Figure 7B is a top view of the components of the optical assembly 200 shown in Fig. 7A.
Figure 8A is a first top view of a portion of the optical assembly 200 of the example embodiment.
Figure 8B is a second top view of the portion of the optical assembly 200 shown in Fig. 8A but with the supporting portion 240 and the arm 220 not being shown.

### [Detailed Description of Example Embodiments]

In view of the background discussed above, the inventors have devised a turret module which at least partly addresses the above-described issues with light beam misalignment in conventional turret modules. More particularly, the inventors have devised a turret module wherein a reflective element (e.g. a mirror) is mounted on an arm that extends into a housing of the turret module, and wherein an adjustable attachment mechanism is provided to attach the arm to the housing and allow an orientation and/or position of the reflective element within the turret module to be adjusted so as to cause the light beam reflected from the reflective element to deviate less from (and preferably propagate along) the optical path. The variability in the light beam emission direction can thus be at least partially compensated for, with fewer or no adjustments to optical elements in the optical system of the ophthalmic imaging apparatus being required.

Example embodiments of the optical assembly will now be described in detail with reference to accompanying drawings.

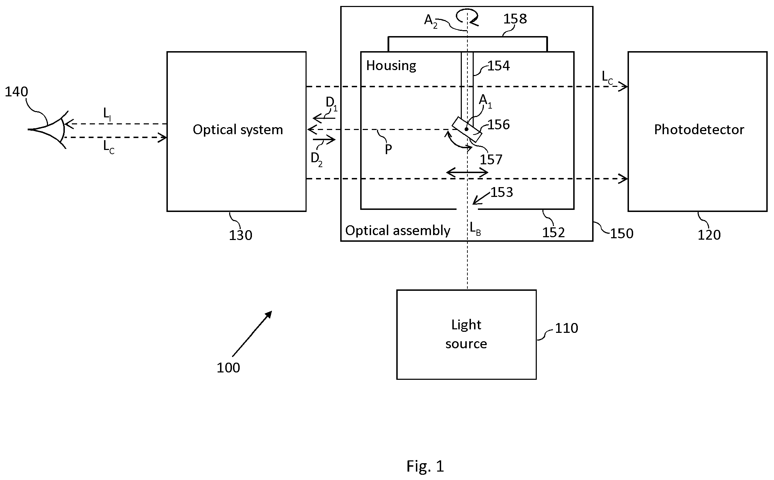

Figure 1 is a schematic illustration of an ophthalmic imaging apparatus 100 according to a first example embodiment herein, which comprises an optical assembly 150. The ophthalmic imaging apparatus 100 further comprises a light source 110 arranged to generate a light beam L_{B}, a photodetector 120, an optical system 130 arranged to illuminate a portion of an eye 140 with light L_{I} from the light beam L_{B} and collect light L_{C} from the illuminated portion of the eye 140. The illuminated portion of the eye 140 may, as in the present example embodiment, comprise a portion of the retina of the eye 140, although the illuminated portion is not so limited and may alternatively comprise another part of the eye 140, such as a portion of the anterior segment, for example.

The optical assembly 150 is arranged to guide the light beam L_{B} from the light source 110 to the optical system 130, and to convey the light L_{C} from the illuminated portion of the eye 140, which has been collected by the optical system 130, towards the photodetector 120. The optical assembly 150 is arranged to convey the collected light L_{C} towards the photodetector 120 via an optical path that includes a first (linear) optical path, P, which passes through the optical assembly 150. As will be described in more detail below, the optical assembly 150 may further comprise one or more optical elements, such as (fold) mirrors, to guide the collected light L_{C} towards the photodetector 120 via the optical path P. The optical assembly 150 may, for example, include an arrangement of mirrors similar to that found in a periscope, which reflects incoming light twice by 90 degrees, using two mirrors that are spaced apart and attached to the housing 152 of the optical assembly (discussed in more detail below), with the optical path P and the reflective element 156 discussed below being disposed between the mirrors. The optical assembly 150 (or "turret module") may, as in the present example embodiment, be detachably mounted in the ophthalmic imaging apparatus 100 so that it can be removed for inspection and maintenance, for example, and then re-installed, as necessary.

The light source 110 is, in general, arranged to generate light in one or more ranges of wavelength that are suitable for imaging the eye 140, for example in the visible spectrum (e.g. red and green light) and/or the near-infrared spectrum. The light source 110 may, for example, comprise one or more laser diodes or super-luminescent diodes (or a combination of laser diodes or super-luminescent diodes), and may also have one or more optical components (such as collimators, apertures, lens) arranged to generate one or more light beams. By way of an example, the light source 110 of the present example embodiment comprises a red laser arranged to generate red light (e.g. of 635 nm wavelength), and a green laser arranged to generate green light (e.g. of 532 nm wavelength), although it will be appreciated that the light source 110 may more generally comprise a first laser arranged to generate light of a first wavelength (defining a first optical channel of the ophthalmic imaging apparatus 100) and a second laser arranged to generate light of a second, different wavelength (defining a second optical channel of the ophthalmic imaging apparatus 100). The two optical channels may, as in the present example embodiment, be combined in a single beam of the light L_{B}, which propagates to the optical system 130 and is used to illuminate the portion of the eye 140. The optical system 130 (discussed further below) may be arranged to illuminate the portion of the eye 140 with a (flying) spot of light or a line of light (in case of the ophthalmic imaging apparatus 100 being a line-field system) generated using a cylindrical lens or other well-known components or optical assemblies for generating line-field illumination.

In example embodiments wherein the ophthalmic imaging apparatus 100 is operable in an optical coherence tomography (OCT) imaging mode, the light source 110 may further comprise a swept light source (in case of the ophthalmic imaging apparatus 100 being a swept-source OCT (SS-OCT) system) or a broadband source (in case of the ophthalmic imaging apparatus 100 being a spectral-domain OCT (SD-OCT) system).

The photodetector 120 may, as in the present example embodiment, comprise a first photodetector (not shown), which is arranged to detect the collected light of the first wavelength, and a second detector (not shown), which is arranged to detect the collected light of the second wavelength. However, the photodetector 120 may instead comprise a single photodetector arranged to detect the collected light of both wavelengths. The photodetector 120 may comprise one or more balanced photodetector arrangements (each comprising two reverse-biased photodiodes whose output photocurrents are subtracted from one another, the subtracted current signal being converted to a voltage detection signal by a transimpedance amplifier). In example embodiments where the ophthalmic imaging apparatus 100 features an OCT imaging modality, the photodetector 120 may further comprise a spectrometer (where a SD-OCT set-up is used) or a photodiode detector (where a SS-OCT set-up is used).

The optical system 130 may, as in the present example embodiment, comprise a scanning system, which is arranged to perform a two-dimensional point-scan of the light L_{I} from the light source 110 across a portion of the eye 140 that is illuminated by the point-scan, and to collect light from the illuminated region during the point-scan.

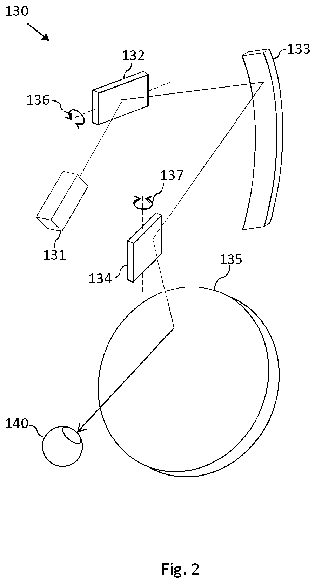

An example of such an optical system is illustrated in Fig. 2. The optical system 130 may, as in the present example embodiment, comprise a scanning element and a mirror, wherein the optical system 130 is arranged to perform the two-dimensional point scan by the scanning element scanning a light beam across the portion of the eye 140 via the mirror. An example of such a scanning system, which can perform a wide-field retinal scan, is described in WO 2014/53824 A1.

Components of such a scanning system are shown in Fig. 2 and comprise an optical coupler 131, a first scanning element 132, a first curved mirror 133, a second scanning element 134 and a second curved mirror 135. The light beam enters the optical system 130 via the optical coupler 131. The light beam is then reflected, in sequence, by the first scanning element 132, the first curved mirror 133, the second scanning element 134 and the second curved mirror 135, before being incident on the eye 140. Light from the illuminated portion of the eye 140 collected by the optical system 130 follows the same optical path through the optical system 130 as the light beam from the optical coupler 131 but in reverse order, and exits the optical system 130 via the optical coupler 131.

The two-dimensional point scan is performed by the first scanning element 132 rotating around a first axis 136 to scan the light beam in a first direction across the portion of the eye 140, and by the second scanning element 134 rotating around a second axis 137 to scan the light beam in a second direction across the portion of the eye 140 (which may, as in the present example embodiment, be orthogonal to the first direction). Thus, by rotating the first scanning element 132 and the second scanning element 134, it is possible to steer the light beam to different locations on the portion of the eye 140. The rotation of the first scanning element 132 and the second scanning element 134 may be coordinated by a scanning system controller (not shown) such that the light beam is scanned across the portion of the eye 140 in accordance with a predefined scan pattern.

In the example of Fig. 2, the first curved mirror 133 is an ellipsoidal mirror (and referred to as a slit mirror), and the second curved mirror 135 is also an ellipsoidal mirror. Each of the ellipsoidal mirrors has a respective first focal point and a respective second focal point. The first scanning element 132 is disposed at the first focal point of the first curved mirror 133, and the second scanning element 134 is disposed at the second focal point of the first curved mirror 133. The second scanning element 134 is also disposed at the first focal point of the second curved mirror 135, and the eye 140 (more specifically, the pupil of the eye 140 in the present example) is disposed at the second focal point of the second curved mirror 135.

The first scanning element 132 and the second scanning element 134 may, as in the present example embodiment, each be a galvanometer optical scanner (or "galvo"), although another type of scanning element could alternatively be used, such as a MEMS scanning mirror or a resonant scanning mirror, for example.

An optical system 130 of the kind described above with reference to Fig. 2 may be used in a variety of imaging modes, such as colour, red-free, autofluorescence (AF), indocyanine green angiography (ICG angiography) and OCT (among others). However, the optical system 130 may alternatively be arranged in other ways, for example to instead perform a line-field scan across the imaged portion of the eye 140. The optical system 130 may further comprise additional optical components, such as optical components for filtering, guiding and/or collimating the light L_{B} from the light source 110, and a cylindrical lens or other means of generating a line of light in case the optical system 130 is a line-field system. It is also noted that the optical system 130 need not be a mirror-based optical system, comprising the first curved mirror 133 and the second curved mirror 135 for focusing and guiding the light beam to the eye 140 but may alternatively be a lens-based optical system comprising an arrangement of lenses to provide these functions.

Referring again to Fig. 1, the optical assembly 150 comprises a housing 152, through which the light beam L_{B} from the light source 110 propagates towards the optical system 130 in a first direction, D₁, along the optical path P during use of the ophthalmic imaging apparatus 100, and through which the light L_{C} collected from the illuminated portion of the eye 140 by the optical system 130 propagates towards the photodetector 120 in a second direction, D₂, along the optical path P during use of the ophthalmic imaging apparatus 100, wherein the second direction D₂ is opposite to the first direction D₁. The housing 152 comprises an opening 153 for allowing the light beam L_{B} to enter the housing 152. The housing 152 is constructed of an opaque material and is arranged not to admit light into the interior thereof, other than via openings in the housing, comprising the opening 153, an opening for receiving the collected light L_{C} from the optical system 130, an opening at the opposite end of the housing 152 (in the direction of the optical path P), via which the collected light L_{C} propagates towards the photodetector 120, and optionally via one or more resealable inspection holes. The shape of the housing 152 is not limited, and may be cylindrical (with the axis of the cylinder lying along the optical axis P) and/or a cuboid, for example.

The optical assembly 150 further comprises an arm 154, which extends into the housing 152. The arm 154 may, as in the present example embodiment, be formed of a rigid material, such as a metal (e.g. steel or aluminium). Furthermore, the arm 154 may, as in the present example embodiment, be formed to have a small profile when viewed along the direction of the optical path P, in order to obstruct as little of the collected (return) light L_{C} propagating along the optical path P (in direction D₂) as possible. The portion of the arm 154 extending into the housing 152 may, as in the present example embodiment, have a projection on a plane perpendicular to the optical path P which has an area that is less than 10 % (more preferably, less than 5 %) of an area of an internal cross-section of the housing 152 where the arm 154 is located or an area of a cross-section of the beam of light L_{C} collected by the optical system 130 that propagates through the housing 152 during use of the ophthalmic imaging apparatus 100. The arm 154 is preferably made to have as thin a profile as possible (i.e. when viewed along the optical path P) while maintaining sufficient rigidity to allow the direction of the light beam L_{B} reflected by the reflective element 156 discussed below to remain stable enough during use of the ophthalmic imaging apparatus 100 to not adversely affect image quality.

The optical assembly further comprises a reflective element 156, which is mounted on (and preferably at an end of) the arm 154 using any suitable means, such as an adhesive, for example. The reflective element 156 comprises a reflective surface 157 for reflecting the light beam L_{B} from the light source 110, which passes through the opening 153 during use of the ophthalmic imaging apparatus 100. The reflective element 156 may take the form of a prism, which reflects a portion of the light beam L_{B} incident on a (partially) reflective surface thereof, or more preferably a plane mirror which reflects substantially all the incident light, for example. It is noted that the reflective element 156 need not have a plane reflective surface, and may have a curvature along one or more axes, if required.

The optical assembly 150 further comprises an adjustable attachment mechanism 158, which is arranged to attach the arm 154 to the housing 152 and to allow adjustment of the arm 154 such that the reflective element 156 mounted on the arm 154 can be adjusted by one or more of: (i) rotating the reflective element 156 about a first axis of rotation, A₁, which passes through a point on the reflective surface 157, the first axis of rotation A₁ being perpendicular to the optical path P; (ii) rotating the reflective element 156 about a second axis of rotation, A₂, which passes through the point on the reflective surface 157, wherein the second axis of rotation A₂ is perpendicular to both the first axis of rotation A₁ and the optical path P; and (iii) translating the reflective element 156 along the optical path P. These one or more adjustments can be used to orientate and/or translate the reflective element 156 such that the reflective element 156 is arranged to reflect the light beam L_{B} to propagate along the optical path P in the first direction D₁ during use of the ophthalmic imaging apparatus 100, thereby at least partially compensating for any misalignments of the light beam L_{B} that would prevent the light beam reflected from the reflective element 156 from propagating along the optical path P. The optical path P may be set to optimise the performance of the ophthalmic imaging apparatus by using, as a performance metric, an aspect of image quality of the acquired image (e.g. absence of clipping, degree of image distortion, etc.), the prevalence of unwanted reflections and/or stray light, a degradation of the spot size of the light beam at the eye, for example.

The light source 110 may, as in the present example embodiment, be slidably mounted within the ophthalmic imaging apparatus 100 (e.g. on a frame of the ophthalmic imaging apparatus 100, which also supports the photodetector 120, the optical system 130 and the optical assembly 150) such that a location on the reflective surface 157, at which the light beam L_{B} generated by the light source 110 is incident on the reflective surface 157, is adjustable along an axis which is perpendicular to the optical path P. Thus, the light source 110 may be slidably mounted on the frame of the ophthalmic imaging apparatus 100 such that the position of the light source 110 along the axis A₂, and therefore the position, along the axis A₂, of the reflected light beam L_{B} propagating in direction D₁ along the optical path P, can be adjusted.

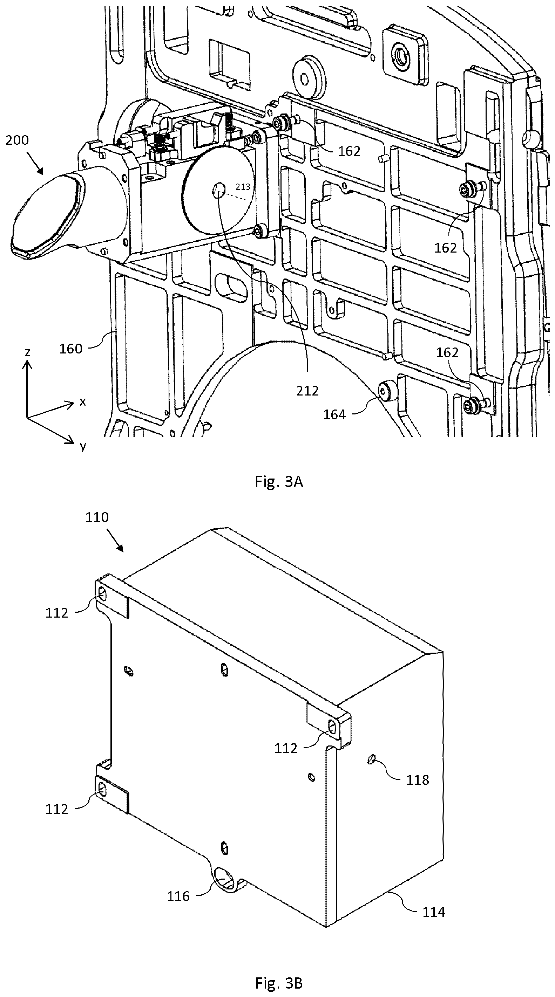

An example of such a slidable mounting of the light source 110, which is employed in the ophthalmic imaging apparatus 100 of the present example embodiment, is illustrated in Figs 3A and 3B. The light source 110 may, as in the present example embodiment, be mounted on a frame 160 of the ophthalmic imaging apparatus 100 via a plurality of slots 112 that are provided in a casing (housing) 114 of the light source 110 and extend in a direction along the rotational axis A₂ (in the z-axis direction) when the light source 110 is mounted on the frame 160. The slots 112 engage with respective (rod-like) protrusions 162 (e.g. dowels and/or screws) that protrude from the frame 160. A cam 164 is attached to the frame 160, and is arranged to engage with the casing 114 of the light source 110, for example via a slot 116 in the casing 114, as shown in Fig. 3B. Rotating the cam 164 (using a key tool or the like) causes the casing 114 of the light source 110 to move up and down along the z-axis. Figure 3B also illustrates an opening 118 in the casing 114 of the light source 110, via which the light beam L_{B} travels into an opening 212 of a housing 210 of the optical assembly 200, which is an example implementation of the optical assembly 150 described in more detail below.

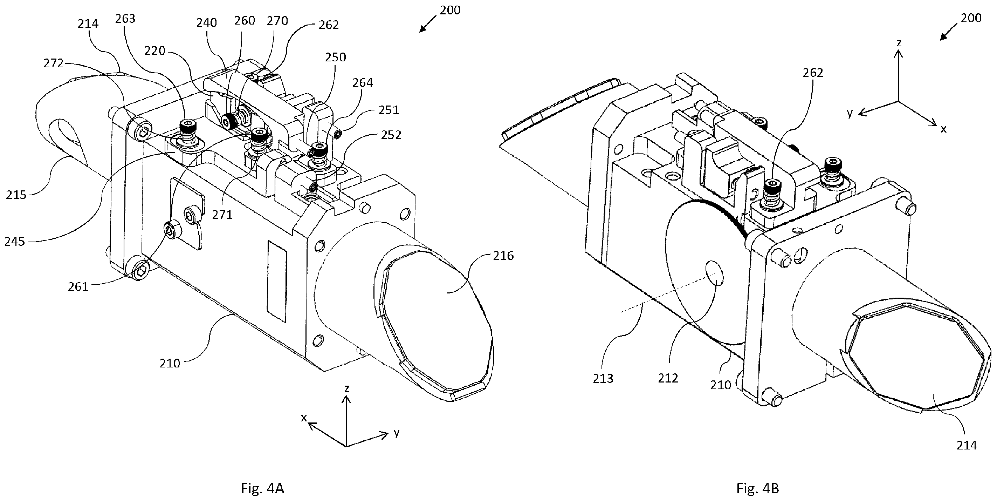

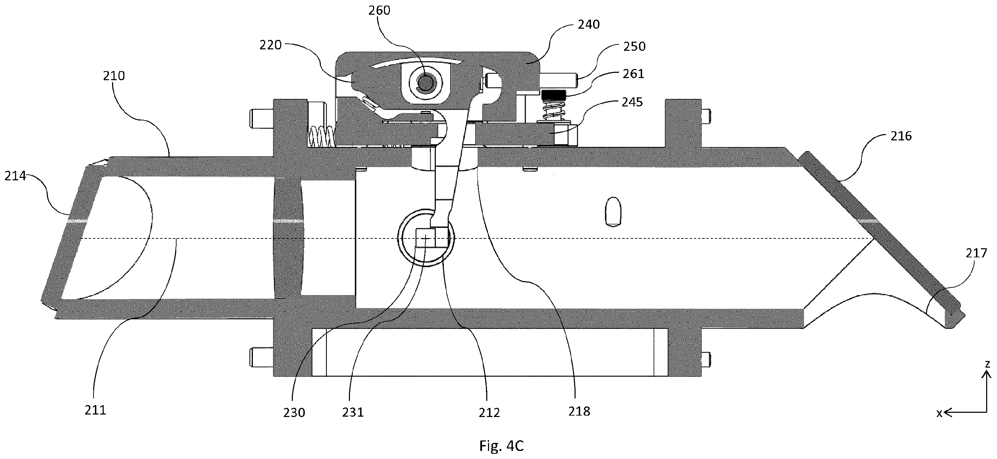

Figures 4A, 4B and 4C are a first isometric view, a second isometric view and a cross-sectional view, respectively, of an optical assembly 200 forming part of an example embodiment The optical assembly 200 is an example implementation of the optical assembly 150 described above with reference to Fig. 1. The housing 210, the arm 220, the prism mirror 230 and an adjustable attachment mechanism comprising supporting portion 240 and base portion 245 illustrated in Figs. 4A to 4C are example implementations of the housing 152, the arm 154, the reflective element 156 and the adjustable attachment mechanism 158 described above. The features and variations of the housing 152, the arm 154, the reflective element 156 and the adjustable attachment mechanism 158, which have been described above, apply to the example implementations of these components that will now be described in more detail.

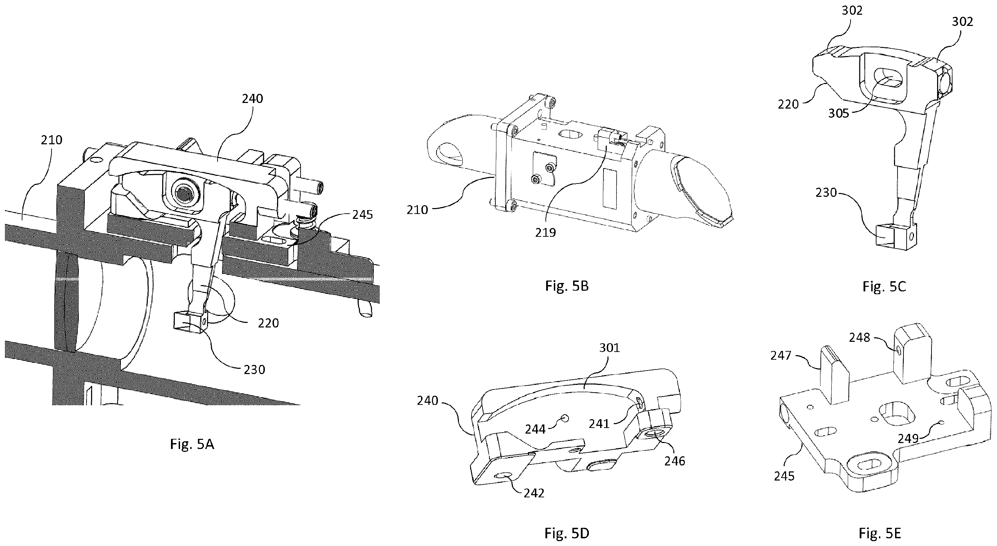

Figure 5A is a cut-away illustration of a part of the optical assembly 200, which includes the housing 210, the arm 220, the prism mirror 230, and portions of the adjustable attachment mechanism including the supporting portion 240 and the base portion 245. These components are further illustrated in the respective isometric views of Figs. 5B to 5E.

The adjustable attachment mechanism may, as in the present example embodiment, comprise the supporting portion 240 and the base portion 245 as separate parts, as illustrated in Figs. 5D and 5E, although the supporting portion 240 and the base portion 245 may alternatively be portions of a single-piece item, as described below.

As illustrated in Fig. 4C, the optical assembly 200 comprises the housing 210, through which the light beam L_{B} from the light source 110 propagates towards the optical system 130 in a first direction along an optical path 211 during use of the ophthalmic imaging apparatus 100, and through which the light L_{C} collected by the optical system 130 propagates towards the photodetector 120 in a second direction along the optical path 211 during use of the ophthalmic imaging apparatus 100, the second direction being opposite to the first direction. In other words, the housing 210 is arranged to enclose an optical path 211 (which is parallel to the x-axis, in a reference coordinate system of the optical assembly 150), along which the light beam L_{B} from the light source 110 propagates towards the optical system 130 in the first direction (i.e. in the positive x-axis direction), and along which the light collected by the optical system 130, L_{C}, propagates towards the photodetector 120 in the second, opposing direction (i.e. in the negative x-axis direction).

As illustrated in Fig. 4B, the housing 210 comprises an opening 212 for allowing the light beam L_{B} generated by the light source 110 to enter the housing 210. The opening 212 may, as in the present example embodiment, be in a side of the housing 210 that extends along the optical path 211. The light source 110 may, as in the present example embodiment, be arranged to emit the light beam L_{B} into the housing 210 along an optical path which is substantially aligned with an entry axis 213 of the housing 210, where the entry axis 213 is perpendicular to the optical path 211. However, owing to achievable manufacturing tolerances of the light source 110, the light beam L_{B} emitted thereby is typically inclined (at a small angle, typically less than 3 degrees) with respect to the entry axis 213 and/or is laterally displaced from the entry axis 213 where light beam L_{B} enters the housing 210. Any such misalignment and/or lateral displacement would cause the light beam reflected by the prism mirror 230 to deviate from the optical path 211.

The housing 210 may, as in the present example embodiment, comprise a cuboidal central portion and two tubular end portions, with one surface of the cuboidal central portion providing a mounting surface for the adjustable attachment mechanism of the optical assembly 200. The optical assembly 200 may, as in the present example embodiment, further comprise a first fold mirror 214 provided at the end of one of the tubular end portions, and a second fold mirror 216 provided at the end of the other tubular end portion. The first fold mirror 214 and the second fold mirror 216 are arranged to reflect light propagating along the optical path 211 through a first fold hole 215 and a second fold hole 217, respectively, thus guiding the light through the optical assembly 200. During operation of the ophthalmic imaging apparatus 100, the light beam L_{B} from the light source 110, which has been reflected by the mirrored surface of the prism mirror 230, exits the housing 210 through the first fold hole 215 as it propagates towards the optical system 130. The light L_{C} collected by the optical system 130 enters the optical assembly 200 through the first fold hole 215, and propagates along the optical path 211 before exiting the housing through the second fold hole 217 and propagating towards the photodetector 120. However, it will be appreciated that one or both of the first fold mirror 214 and the first fold hole 215, and the second fold mirror 216 and the second fold hole 217, may be omitted, and the light propagating along the optical path 211 may simply be conveyed (or guided using one or more differently arranged mirrors) towards the optical system 130 or the photodetector 120 (as the case may be), depending on how the photodetector 120 and the optical system 130 are arranged relative to each other in the ophthalmic imaging apparatus 100.

Referring to Fig. 4C, the arm 220 extends into the housing 210. The arm 220 may, as in the present example embodiment, extend through a second opening 218 in housing 210. The portion of the arm 220 extending into the housing 210 may, as in the present example embodiment, have a projection on a plane perpendicular to the optical path P which has an area that is less than 10 % (more preferably, less than 5 %) of an area of a cross-section of the housing 210 where the arm 220 is located or an area of a cross-section of the light L_{C} collected by the optical system 130 that propagates through the housing 210 during use of the ophthalmic imaging apparatus 100. The arm 220 is preferably made to have a thin a profile as possible (when viewing along the direction of the optical path 211) while maintaining sufficient rigidity to allow the direction of the light beam L_{B} reflected by the prism mirror 230 to remain stable enough during use of the ophthalmic imaging apparatus 100 to not adversely affect image quality. By minimising the area of the aforementioned projection (profile) of the arm 220, as little of the collected light L_{C} as possible is prevented by the arm 220 from propagating towards the photodetector 120, thereby helping to improve signal-to-noise and thus the imaging quality.

The prism mirror 230 is mounted on the arm 220 and comprises a glass prism having a silvered side that provides a reflective surface. The prism mirror 230 is mounted at the end of the arm 220, with the reflective surface being orientated to reflect the light beam L_{B} incident thereon via the opening 218 during use of the ophthalmic imaging apparatus 100, such that the reflected beam travels along the housing 210, towards the first fold mirror 214 and subsequently towards the optical system 130, via the first fold hole 215. It is noted that, in other example embodiments, the prism mirror 230 may be replaced with an alternative form of reflective element, such as a plane mirror, which may be mounted on the arm 220 at an appropriate angle to reflect the incident light beam L_{B} towards the optical system 130.

Although the entry axis 213 shown in Fig. 4B is parallel to the y-axis in the present example embodiment, the entry axis 213 may alternatively be parallel to the z-axis in an alternative example embodiment, with the opening 212 correspondingly being provided in the bottom side of the central (cuboidal) portion of the housing 210, i.e. in the opposite surface of the housing 210 to the mounting surface for the adjustable attachment mechanism of the optical assembly 150. In this case, the prism mirror 230 would be appropriate angled to reflect the light beam L_{B} entering the housing 210 towards the optical system 130.

The adjustment attachment mechanism is arranged to attach the arm 220 to the housing 210 (as described in more detail below), and to allow prism mirror 230 to be adjusted by at least one of (i) rotating the prism mirror 230 about a first axis of rotation (parallel to the y-axis in Fig. 4C), which passes through a point 231 on the reflective surface, (ii) rotating the prism mirror 230 about a second axis of rotation (parallel to the z-axis in Fig. 4C), which passes through the point 231 and is perpendicular to the first axis of rotation, and (iii) translating the prism mirror 230 along the optical path 211, such that, during use of the ophthalmic imaging apparatus 100, the prism mirror 230 is arranged to reflect the light beam L_{B} incident on the reflective surface thereof to propagate along the optical path 211 in the first direction (D₁ in Fig. 1), towards the optical system 130.

Accordingly, the orientation and/or position of the prism mirror 230 can be adjusted by adjusting the orientation and/or position of the arm 220 in the optical assembly such that the prism mirror 230 reflects the light beam L_{B} in the first direction D₁ along the optical path 211, thereby compensating for a deviation in the direction of travel of the light beam L_{B} from the entry axis 213. Such deviation, if not compensated for by appropriate adjustment of the adjustable attachment mechanism to produce the necessary change in the orientation and/or position of the prism mirror 230 along the optical axis 211, would result in the light beam reflected by the prism mirror 230 not propagating along the optical axis 211.

The first axis of rotation A₁ may, as in the present example embodiment, be perpendicular to the optical path 211 and parallel to the entry axis 213 (i.e. the y-axis direction), although it may be any axis of rotation which passes the point 231 and is perpendicular to, or has at least a directional component perpendicular to, the optical path 211. The second axis of rotation A₂ may, as in the present example embodiment, be perpendicular to the first axis of rotation A₁ and the optical path 211 (i.e. the second axis of rotation A₂ may be in the z-axis direction), although the second axis of rotation A₂ may instead be any axis which at least has directional components perpendicular to the optical axis 211 and the first axis of rotation A₁.

The point 231 on the reflective surface of the prism mirror 230 may, as in the present example embodiment, be the geometric centre of the reflective surface, although it may, more generally, be any point on the reflective surface. The adjustment attachment mechanism may, as in the present example embodiment, be arranged to allow the prism mirror 230 to be adjusted by all three of the aforementioned degrees of freedom. However, in other example embodiments, the adjustment attachment mechanism may allow the prism mirror 230 to be adjusted by only one or only two of the aforementioned degrees of freedom.

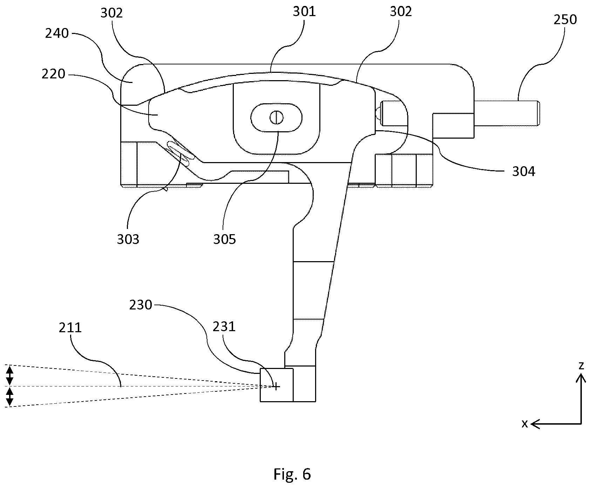

The adjustable attachment mechanism may, as in the present example embodiment, be arranged to allow the prism mirror 230 mounted on the arm 220 to be adjusted by rotating the prism mirror 230 about the first axis of rotation A₁. This rotation may, as in the present example embodiment, be achieved by providing the adjustable attachment mechanism with a first contact surface, as shown at 301 in Figs. 5D and 6, where the first contact surface 301 is, by way of example, provided on the supporting portion 240 of the adjustable attachment mechanism. The first contact surface 301 may, however, more generally be provided on any component of the adjustable attachment mechanism whose displacement from the point 231 is fixed. The first contact surface 301 faces the reflective element 230, and has the shape of a part of a surface of revolution about the first axis of rotation A₁ (which is parallel to the y-axis in Fig. 6). Furthermore, to enable the rotation of prism mirror 230 about the first axis of rotation A₁, the arm 220 has one or more second contact surfaces which contact the first contact surface 301 at points that are equidistant from the first axis of rotation A₁ and have different respective angular positions about the first axis of rotation A₁. The arm 220 may, as in the present example embodiment have two such second contact surfaces, which are shown at 302 in Figs. 5C and 6, although the number of second contact surfaces 302 is not so limited and may be more than two in other example embodiments. Each second contact surface 302 may have the shape of a part of a surface of revolution about the first axis of rotation A₁, as in the present example embodiment. It should be noted, however, that the arm may alternatively have only one second contact surface, which contacts the first contact surface 301 at points that are equidistant from the first axis of rotation A₁ and have different respective angular positions about the first axis of rotation A₁, the second contact surface of the arm in that case having the shape of a part of a surface of revolution about the first axis of rotation A₁. The second contact surfaces 302 face and contact the first contact surface 301, as shown in Fig. 6, and each of the second contact surfaces 302 has the shape of the part of the surface of revolution about the first axis of rotation A₁, i.e. substantially the same radius of curvature as the first contact surface 301. The second contact surfaces 302 are slidable over the first contact surface 301 such that the prism mirror 230 rotates about the first axis of rotation A₁. The second contact surfaces 302 are thus slidable over the first contact surface 301 and, as the opposing contact surfaces remain in contact while sliding over one another, the arm 220 is caused to rotate about the common axis of the surface of revolution, i.e. the first axis of rotation A₁.

Figure 6 is a side view of some components of the optical assembly 200, including the supporting portion 240 of the adjustable attachment mechanism, and the arm 220, as well as some further components that are discussed below. As shown in Fig. 6, the supporting portion 240 of the adjustable attachment mechanism has the first contact surface 301, and the arm 220 has the second contact surfaces 302, and the first and second contact surfaces 301 and 302 have a common shape of a part of a surface of revolution about an axis of rotation which is along the y-axis in Fig. 6 and passes through the point 231 on the reflective surface of the prism mirror 230. The surface of revolution may, as in the present example embodiment, be a cylinder. Each of the first contact surface 301 and second contact surfaces 302 thus has a curvature in the x-z plane which is a part of the circumference of a common circle centred on the point 231. However, the surface of revolution is not so limited and alternatively be frustoconical, for example. The perpendicular distance of the surface of revolution from the axis of rotation may vary non-monotonically along the axis of rotation.

The rotation of the prism mirror 230 about the first axis of rotation A₁ that is enabled by the adjustment mechanism is illustrated in Fig. 6 relative to the shown portion of the optical path 211 by the angled dashed lines.

The optical assembly 150 may, as in the present example embodiment, further comprise a first moveable member 250 attached to the adjustable attachment mechanism and having an end abutting a side 304 of the arm 220. The optical assembly may, as shown in Fig. 6, further comprise a first resilient member 303, which is arranged to force the side 304 of the arm 220 against the end of the first moveable member 250. The first moveable member 250 is moveable relative to the adjustable attachment mechanism to vary a position of the end of the first moveable member 250 relative to the adjustable attachment mechanism. The first moveable member 250, when moved relative to the adjustable attachment mechanism, is arranged to cause the arm 220 to move such that the second contact surfaces 302 slide over the first contact surface 301, with the arm 220 and the prism mirror 230 mounted thereon consequently rotating about the first axis of rotation A₁, which passes through the point 231 on the reflective surface of the prism mirror 230. The first resilient member 303 may, as in the present example embodiment, further be arranged to force the second contact surfaces 302 of the arm 220 against the first contact surface 302 of the supporting portion 240. However, in other example embodiments, the second contact surfaces 302 may be forced against the first contact surface 302 by another resilient member, instead of the first resilient member 303.

The first resilient member 303 may, as in the present example embodiment, comprise a helical compression spring, which is held in compression between a recess in the supporting portion 240 that houses an end of the spring, and a recess in a surface of the arm 220, which houses the other end of spring. However, any other kind of resilient member know to those versed in the art may be used, such as a torsion spring or a flexure, which may be made of a steel alloy (such as spring steel) or other material having a high yield strength. The first resilient member 303 may alternatively comprise rubber or the like.

The first moveable member 250 may, as in the present example embodiment, comprise a first screw, which has been screwed through a first threaded portion of the adjustable attachment mechanism (specifically, a threaded hole 241 in the supporting portion 240, as illustrated in Fig. 5D), wherein an end of the first screw abuts the side 304 of the arm 220. The side 304 of the arm 220 may comprise a plate of sapphire or another hard material that is resistant to indentation by the end of the first screw. The first screw, when rotated to move through the threaded hole 241, causes the arm 220 to move such that the second contact surfaces 302 of the arm 220 slide over the first contact surface 301.

The form of the first moveable member 250 is not limited in this way, however. For example, a cam which is attached to the adjustable attachment mechanism and has an end abutting the side 304 of the arm 220, with the first resilient member 303 forcing the side 304 of the arm 220 against the end of the cam, may be used in place of the first screw. In this variant, the cam can be rotated relative to the adjustable attachment mechanism to vary the position of the end of the cam relative to the adjustable attachment mechanism. The cam, when rotated, is arranged to cause the arm 220 to move such that the second contact surfaces 302 of the arm 220 slide over the first contact surface 301.

Referring to Fig. 4A, the arm 220 may, as in the present example embodiment, be biased (forced) against the supporting portion 240 by a first attachment screw 260, which has been screwed into a threaded hole 244 of the supporting portion 240, and a first attachment resilient member 270 (in the form of a helical compression spring, for example), which is held in compression between the head of the first attachment screw 260 and the surface of the arm 220 that faces the head of the first attachment screw 260 (optionally via intermediary washer(s)). The first attachment screw 260 passes through an elongated hole or slot 305 in the arm 220 (as shown in Figs. 5C and 6) so that it does not impede the above-described rotation of the prism mirror 230 about the first axis of rotation A₁. However, the arm 220 may be biased against the supporting portion 240 by other means in other example embodiments. For instance, in an example embodiment wherein each of the first contact surface 301 and the second contact surface(s) 302 is shaped as part of a frustoconical surface of revolution about the first axis of rotation A₁, with the radius of the surface of revolution increasing along the negative y-axis direction, the arm 220 may be biased against the supporting portion 240 by the first resilient member 303, as the first contact surface 301 effectively wedges the arm 220 against the supporting member 240.

Although the present example embodiment uses two contact surfaces which slide over one another to provide the rotation of the prism mirror 230 about the first axis of rotation A₁, others means of achieving such rotation are possible. For example, in an alternative example embodiment, the arm 154 comprises two arcuate slots that run along a common arc of a circle centred on the first rotational axis A₁. A screw passes through each of these arcuate slots and is screwed into a respective threaded hole in the supporting portion of the adjustable attachment mechanism. The diameter of the screws is chosen to be similar to the width of the arcuate slots. Thus, in this alternative example embodiment, the arm is constrained by the screws to move along the arc of the circle such that the rotatable element 230 can be rotated about the first rotational axis A₁ and, when the desired rotation has been achieved, the screws can be tightened such that they fix the arm to the supporting portion of the adjustable attachment mechanism. The screws may subsequently be loosened to re-adjust the rotation of the prism mirror 230, when required.

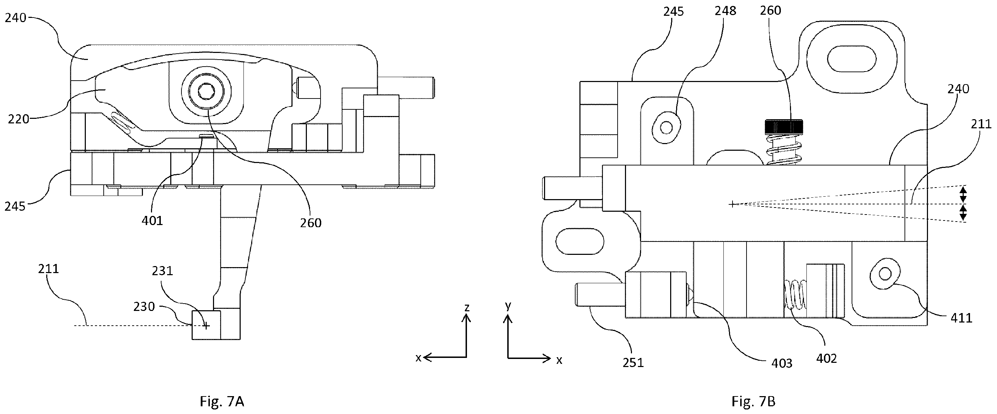

The adjustable attachment mechanism may, as in the present example embodiment, further be arranged to allow the prism mirror 230 mounted on the arm 220 to be adjusted by rotating the prism mirror 230 about the second axis of rotation A₂. This rotation may, as in the present example embodiment, and as illustrated in Figs. 7A and 7B, be achieved by arm 220 being attached to the supporting portion 240 of the adjustable attachment mechanism (as previously described), and the supporting portion 240 being rotatable about a pivot 401 which is aligned with the second axis of rotation A₂ such that the prism mirror 230 is rotatable about the second axis of rotation A₂. The supporting portion 240 is therefore able to rotate around the pivot 401 and, as the arm 220 is attached to the supporting portion 240 and supports the prism mirror 230 such that the point 231 on the reflective surface of the prism mirror 230 lies on the second axis of rotation A₂, the prism mirror 230 is caused to rotate about the second axis of rotation A₂.

Figure 7A is a side view of some components of the optical assembly 200, including the supporting portion 240 and base portion 245 of the adjustable attachment mechanism, the arm 220, and the pivot 401. Figure 7B is a top view of the components of the optical assembly 200 shown in Fig. 7A.

As illustrated, the pivot 401 may, as in the present example embodiment, be provided on the base portion 245 of the adjustable attachment mechanism. The pivot 401 may, as in the present example embodiment, be provided in the form of a dowel pin, which is received in holes in the supporting portion 240 and the base portion 245. However, any other suitable kind of pivot may be used, such as a taper pin, for example. The rotation of the prism mirror 230 about the second axis of rotation A₂ that is enabled by the adjustment mechanism is illustrated by the dashed lines in Fig. 7B that are angled relative to the optical path 211.

The optical assembly 150 may, as in the present example embodiment, further comprise a second moveable member 251, which is attached to the adjustable attachment mechanism (to a portion of the adjustable attachment mechanism other than the supporting portion 240) and has an end abutting a side 403 of the supporting portion 240, as illustrated in Fig. 7B, and further comprise a second resilient member 402, which is arranged to force the side 403 of the supporting portion 240 against the end of the second moveable member 251. The second moveable member 251, when moved relative to the adjustable attachment mechanism, is arranged to cause the supporting portion 240 to rotate about the pivot 401, thereby causing the prism mirror 230 to rotate about the second axis of rotation A₂.

The second resilient member 402 may, as in the present example embodiment, comprise a helical compression spring, which is held in compression between a recess in a protruding part 247 of the base portion 245 (as shown in Fig. 5E), which houses an end of the spring, and a recess in a surface of the supporting portion 240, which houses the other end of spring. However, any other kind of resilient member know to those versed in the art may be used, such as a torsion spring or a flexure, which may be made of a steel alloy (such as spring steel) or other material having a high yield strength. The second resilient member 402 may alternatively comprise rubber or the like.

The second moveable member 251 may, as in the present example embodiment, comprise a second screw, which has been screwed through a second threaded portion of the adjustable attachment mechanism (specifically, a threaded hole 248 in the base portion 245, as illustrated in Fig. 5E), wherein an end of the second screw abuts the side 403 of the supporting portion 240. The side 403 of the supporting portion 240 may comprise a plate of sapphire or another hard material that is resistant to indentation by the end of the second screw. The second screw, when rotated to move through the threaded hole 248, causes the supporting portion 240 to rotate about the pivot 401, thereby causing the prism mirror 230 to rotate about the second axis of rotation A₂.

The form of the second moveable member 251 is not limited in this way, however. For example, a cam which is attached to the adjustable attachment mechanism and has an end abutting the side 403 of the supporting portion 240, with the second resilient member 402 forcing the side 403 of the supporting portion 240 against the end of the cam, may be used in place of the second screw. In this variant, the cam can be rotated relative to the adjustable attachment mechanism to vary the position of the end of the cam relative to the adjustable attachment mechanism. The cam, when rotated, is arranged to cause the supporting portion 240 to rotate about the pivot 401, thereby causing the prism mirror 230 to rotate about the second axis of rotation A₂.

The supporting portion 240 may, as in the present example embodiment, be attached to the base portion 245. Referring to Figs. 4A and 5E, the supporting portion 240 may, as in the present example embodiment, be biased (forced) against the base portion 245 by a second attachment screw 261, which has been screwed into a threaded hole 249 of the base portion 245, and a second attachment resilient member 271 (in the form of a helical compression spring, for example), which is held in compression between the head of the second attachment screw 261 and the surface of the supporting portion 240 that faces the head of the second attachment screw 261 (optionally via intermediary washer(s)). The second attachment screw 261 passes through an enlarged hole 246 (as shown in Figs. 5D and 7B) so that it does not impede the above-described rotation of the supporting portion 240 about the pivot. However, the supporting portion 240 may be biased against the base portion 245 by other means in other example embodiments.

Additional attachment screws, the same as described above, may be provided to force the supporting portion 240 against the base portion 245, such as attachment screw 262 (shown in Fig. 4B) through enlarged hole 242 in Fig. 5D. Further, the supporting portion 240 can be attached to the base portion 245 by other means, such as by the dowel pin comprising protrusions which fix the position of the supporting portion 240 and the base portion 245 in the z-direction.

Although the present example embodiment employs a pivot 401 to rotate the prism mirror 230 about the second axis of rotation A₂, another means for achieving this rotation may be used instead. For example, the supporting portion 240 and base portion 245 may have respective contact surfaces whose shape is defined by a part of a surface of revolution about the second axis of rotation A₂, in a similar manner to how the above-described rotation of the prism mirror 230 about the first axis of rotation A₁ is achieved in the present example embodiment.

The adjustable attachment mechanism may, as in the present example embodiment, be further arranged to allow the prism mirror 230 mounted on the arm 220 to be adjusted by translating the prism mirror 230 along the optical path 211. This may, as in the present example embodiment, be achieved by the base portion 245 of the adjustable attachment mechanism being slidably attached to the housing 210 so as to be slidable along a direction parallel to the optical path 211, such that the prism mirror 230 mounted on the arm 220 is translatable along the optical path 211.

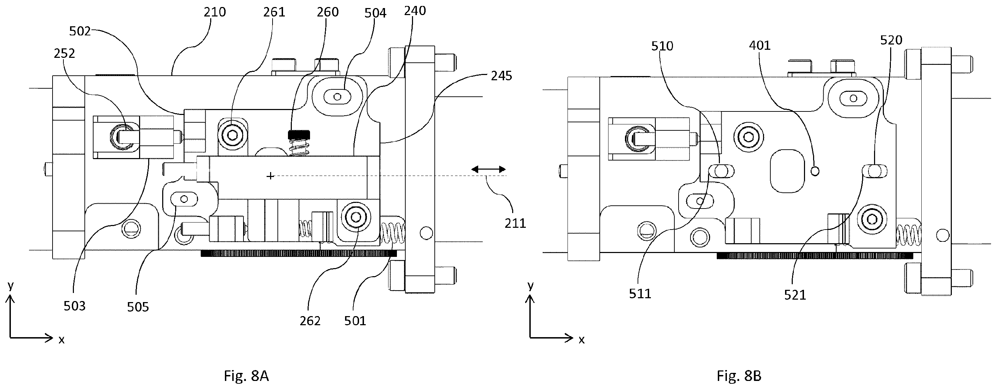

Figure 8A is a first top view of a portion of the optical assembly 200. Figure 8B is a second top view of the portion of the optical assembly 200 shown in Fig. 8A but with the supporting portion 240 and the arm 220 not being shown, to more clearly illustrated the mechanism by which the base portion 245 is slidably attached to the housing 210.

In the present example embodiment, the base portion 245 is constrained to move in the direction of the optical path 211 (i.e. in the x-axis direction) by the presence of two dowel pins 510 and 520, which sit within respective holes 511 and 521, as shown in Fig. 8B. However, the movement of the base portion 245 may be constrained along the x-axis by other means, such as guiding rails on the housing 210 which run along either side of the base portion 245, or by the third attachment screw 263 and/or fourth attachment screw 264 (as described below), for example.

The optical assembly 200 may, as in the present example embodiment, further comprise a third moveable member 252, which is attached to the housing 210 and has an end abutting a side 502 of the base portion 245. The optical assembly 200 may also have a third resilient member 501, which is arranged to force the side 502 of the base portion 245 against the end of the third moveable member 252. When the third moveable member 252 is moved relative to the housing 210, the third moveable member 252 causes the base portion 245 to slide along the housing 210, in the direction parallel to the optical path 211, thereby causing the reflective element 230 mounted on the arm 220 to be translated along the optical path 211. In other words, the third moveable member 252 moves the base portion 245 along the optical path 211 in opposition to a force exerted by the third resilient member 501, thereby moving the prism mirror 230 in the same direction, as the prism mirror 230 is mounted on the arm 220, which is attached to the supporting portion 240 and therefore also the base portion 245.

The third resilient member 501 may, as in the present example embodiment, comprise a helical compression spring, which is held in compression between a recess in the housing 210, which houses an end of the spring, and a recess in an opposing surface of the base portion 245, which houses the other end of spring. However, any other kind of resilient member know to those versed in the art may be used, such as a torsion spring or a flexure made of a steel alloy (such as spring steel) or other material having a high yield strength. The third resilient member 501 may alternatively comprise rubber or the like.

The third moveable member 252 may, as in the present example embodiment, comprise a third screw, which has been screwed through a threaded portion of the adjustable attachment mechanism (specifically, a threaded hole in a post 219, which is attached to the housing 210, as illustrated in Fig. 5B), wherein an end of the third screw abuts the side 502 of the base portion 245. The side 502 of the base portion 245 may comprise a plate of sapphire or another hard material that is resistant to indentation by the end of the third screw. The third screw, when rotated to move through the threaded hole in post 219, causes the base portion 245 to slide over the surface of the housing 210, thereby causing the prism mirror 230 to be translated along the optical path 211.

The form of the third moveable member 252 is not limited in this way, however. For example, a cam which is attached to the top surface of the housing 210 shown in Fig. 5B, and has an end abutting the side 502 of the base portion 245, with the third resilient member 501 forcing the side 502 of the base portion 245 against the end of the cam, may be used in place of the third screw. In this variant, the cam can be rotated relative to the housing 210 to vary the position of the end of the cam relative to the housing 210. The cam, when rotated, is arranged to cause the base portion 245 to slide over the surface of the housing 210, thereby causing the prism mirror 230 to be translated along the optical path 211.

The base portion 245 may, as in the present example embodiment, be slidably attached to the housing 210 by a third attachment screw 263, which is disposed in a threaded portion of the housing 210, and a third attachment resilient member 272, which is held in compression between a head of the third attachment screw 263 and a surface of the base portion 245. The third attachment screw 263 passes through an enlarged hole 504 in the base portion 245, which is sized so as not to impede the above-described translation of the base portion 245, the supporting portion 240, the arm 220 and the prism mirror 230. Additional attachment screws, the same as described above, may be provided between the base portion 245 and the housing 210, such as the fourth attachment screw 264 through hole 505 in the base portion 245 shown in Figs. 4A and Fig. 8A.

The first threaded portion 241, the second threaded portion 248, and the third threaded portion may, as in the present example embodiment, be arranged to extend along a direction parallel to the optical path 211, such that the first screw 250, the second screw 251 and the third screw 252 can each be rotated about respective axes that are parallel to one another. This is illustrated in Fig. 4A, wherein the screws 250, 251, and 252 pass through respective threaded holes that all extend in a common direction which is parallel (or substantially parallel) to the x-axis. Accordingly, a technician would be able to access and adjust all three screws conveniently, using a hex key or the like.

In the present example embodiment, the adjustable attachment mechanism allows the prism mirror 230 to be rotated about the first axis of rotation A₁ and the second axis of rotation A₂, and to also be translated along the optical path 211. To allow adjustment along these three degrees of freedom, the adjustable attachment mechanism is arranged to attach the arm 220 to the housing 210 by the arm 220 being attached to the supporting portion 240, the supporting portion 240 being attached to the base portion 245, and the base portion 245 being slidably attached to the housing 210. However, only one or two of these three degrees of freedom may be provided by the adjustment attachment mechanism in some example embodiments. For example, the supporting portion 240 and the base portion 245 may be merged to form a single part if rotation about the second axis of rotation A₂ is not required, the base portion 245 may be fixed to the housing 210, with no provision to make it movable relative to the housing 210, if the translational movement of the prism mirror 230 along the optical axis 211 is not required, and/or the supporting portion 240 may be merged with the arm 220 to become a part of the arm 220 if rotation of the prism mirror 230 about the first axis of rotation A₁ is not required.

By the adjustable attachment mechanism allowing the prism mirror 230 to be adjustable by a rotation about the first axis of rotation A₁, a rotation about the second axis of rotation A₂, a translation along the optical path 211, and/or a translation in a direction perpendicular to the optical path, the prism mirror 230 may be set to reflect the light beam incident thereon so as to propagate along the optical path 211 for any deviation in the direction of travel of the light beam from the light source 110 from the entry axis 213. For example, referring to Fig. 3A, an offset of the light beam from the entry axis 213 along the x-axis direction would result in the light beam reflected from the prism mirror 230 being correspondingly offset from the optical path 211 in the y-axis direction. By the adjustable attachment mechanism allowing translation of the prism mirror 230 along the optical path 211, the aforementioned offset of the light beam from the entry axis 213 can be corrected to eliminate the consequent offset of the reflected light beam from the optical axis 211.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the example embodiments described above, but should be defined only in accordance with the following claims.

While this specification contains many specific embodiment details, these should not be construed as limitations on the scope of any inventions or of what may be claimed, but rather as descriptions of features specific to particular embodiments described herein.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An ophthalmic imaging apparatus (100), comprising:
a light source (110) arranged to generate a light beam (L_{B});
a photodetector (120);
an optical system (130) arranged to illuminate a portion of an eye (140) with light from the light beam (L_{B}) and collect light (L_{C}) from the illuminated portion of the eye (140); and
an optical assembly (150) arranged to guide the light beam (L_{B}) from the light source (110) to the optical system (130), and to convey the light (L_{C}) collected by the optical system (130) towards the photodetector (120),
the optical assembly (150) comprising:
a housing (152; 210) through which the light beam (L_{B}) from the light source (110) propagates towards the optical system (130) in a first direction (D₁) along an optical path (P; 211) during use of the ophthalmic imaging apparatus (100), and through which the light (L_{C}) collected by the optical system (130) propagates towards the photodetector (120) in a second direction (D₂) along the optical path (P; 211) during use of the ophthalmic imaging apparatus (100), wherein the second direction (D₂) is opposite to the first direction (D₁), and the housing (152; 210) comprises an opening (153) for allowing the light beam (L_{B}) generated by the light source (110) to enter the housing (152; 210);
an arm (154; 220) which extends into the housing (152; 210);
a reflective element (156; 230) mounted on the arm (154; 220), the reflective element (156; 230) comprising a reflective surface (157) for reflecting the light beam passing through the opening (153) during use of the ophthalmic imaging apparatus (100); and
an adjustable attachment mechanism (158) arranged to attach the arm (154; 220) to the housing (152; 210) and to allow the reflective element (156; 230) to be adjusted by at least one of:
rotating the reflective element (156; 230) about a first axis of rotation (A₁) passing through a point (231) on the reflective surface (157), the first axis of rotation (A₁) being perpendicular to the optical path (P; 211);
rotating the reflective element (156; 230) about a second axis of rotation (A₂) passing through the point (231) on the reflective surface (157), the second axis of rotation (A₂) being perpendicular to the first axis of rotation (A₁) and the optical path (P; 211); or
translating the reflective element (156; 230) along the optical path (P; 211),
such that, during use of the ophthalmic imaging apparatus (100), the reflective element (156; 230) is arranged to reflect the light beam (L_{B}) passing through the opening (153) to propagate along the optical path (P; 211) in the first direction (D₁).

2. The ophthalmic imaging apparatus (100) according to Claim 1, wherein
the adjustable attachment mechanism (158) is arranged to allow the reflective element (156; 230) mounted on the arm (154; 220) to be adjusted by rotating the reflective element (156; 230) about the first axis of rotation (A₁), and comprises a first contact surface (301) which faces the reflective element (156; 230), wherein the first contact surface (301) has a shape of a part of a surface of revolution about the first axis of rotation (A₁),
the arm (154; 220) comprises one or more second contact surfaces (302) arranged to contact the first contact surface (301) at points that are equidistant from the first axis of rotation (A₁) and have different respective angular positions about the first axis of rotation (A₁), and
the one or more second contact surfaces (302) are slidable over the first contact surface (301) such that the reflective element (156; 230) is rotatable about the first axis of rotation (A₁).

3. The ophthalmic imaging apparatus (100) according to Claim 2, wherein
the adjustable attachment mechanism (158) further comprises:
a first moveable member (250) attached to the adjustable attachment mechanism (158) and having an end abutting a side (304) of the arm (154; 220), wherein the first moveable member (250) is moveable relative to the adjustable attachment mechanism (158) to vary a position of the end of the first moveable member (250) relative to the adjustable attachment mechanism (158); and
a first resilient member (303) arranged to force the side (304) of the arm (154; 220) against the end of the first moveable member (250), and
the first moveable member (250), when moved relative to the adjustable attachment mechanism (158), is arranged to cause the arm (154; 220) to move such that the one or more second contact surfaces (302) slide over the first contact surface (301).

4. The ophthalmic imaging apparatus (100) according to Claim 3, wherein the first moveable member (250) attached to the adjustable attachment mechanism (158) comprises a first screw which has been screwed through a first threaded portion of the adjustable attachment mechanism (158), wherein an end of the first screw abuts the side (304) of the arm (154; 220), and wherein the first screw, when rotated to move through the first threaded portion, causes the arm (154; 220) to move such that the one or more second contact surfaces (302) slide over the first contact surface (301).

5. The ophthalmic imaging apparatus (100) according to any one of the preceding claims, wherein the adjustable attachment mechanism (158) allows the reflective element (156; 230) mounted on the arm (154; 220) to be adjusted by rotating the reflective element (156; 230) about the second axis of rotation (A₂), and comprises a supporting portion (240) attached to the arm (154; 220), the supporting portion (240) being rotatable about a pivot (401) which is aligned with the second axis of rotation (A₂) such that the reflective element (156; 230) mounted on the arm (154; 220) is rotatable about the second axis of rotation (A₂).

6. The ophthalmic imaging apparatus (100) according to Claim 5, wherein
the adjustable attachment mechanism (158) comprises:
a second moveable member (251) attached to the adjustable attachment mechanism (158) and having an end abutting a side (403) of the supporting portion (240); and
a second resilient member (402) arranged to force the side (403) of the supporting portion (240) against the end of the second moveable member (251), and
the second moveable member (251), when moved relative to the adjustable attachment mechanism (158), is arranged to cause the supporting portion (240) to rotate about the pivot (401), thereby causing the reflective element (156; 230) to rotate about the second axis of rotation (A₂).

7. The ophthalmic imaging apparatus (100) according to Claim 6, wherein the second moveable member (251) attached to the adjustable attachment mechanism (158) comprises a second screw which has been screwed through a second threaded portion of the adjustable attachment mechanism (158), wherein an end of the second screw abuts the side (403) of the supporting portion (240), and wherein the second screw, when rotated to move through the second threaded portion, causes the supporting portion (240) to rotate about the pivot (401).

8. The ophthalmic imaging apparatus (100) according to any one of the preceding claims, wherein the adjustable attachment mechanism (158) allows the reflective element (156; 230) mounted on the arm (154; 220) to be adjusted by translating the reflective element (156; 230) along the optical path (P; 211), the adjustable attachment mechanism (158) comprising a base portion (245) which is slidably attached to the housing (152; 210) so as to be slidable along the housing (152; 210), in a direction parallel to the optical path (P; 211), such that the reflective element (156; 230) mounted on the arm (154; 220) is translatable along the optical path (P; 211).

9. The ophthalmic imaging apparatus (100) according to Claim 8, wherein the optical assembly further comprises:
a third moveable member (252) attached to the housing (152; 210) and having an end abutting a side (502) of the base portion (245); and
a third resilient member (501) arranged to force the side (502) of the base portion (245) against the end of the third moveable member (252),
wherein the third moveable member (252), when moved relative to the housing (152; 210), is arranged to cause the base portion (245) to slide along the housing (152; 210), in the direction parallel to the optical path (P; 211), thereby causing the reflective element (156; 230) mounted on the arm (154; 220) to be translated along the optical path (P; 211).

10. The ophthalmic imaging apparatus (100) according to Claim 9, wherein the third moveable member (252) attached to the housing (152; 210) comprises a third screw which has been screwed through a third threaded portion of the adjustable attachment mechanism (158), wherein an end of the third screw abuts the side (502) of the base portion (245), and wherein the third screw, when rotated to move through the third threaded portion, causes the base portion (245) to slide along the housing (152; 210), in the direction parallel to the optical path (P; 211).

11. The ophthalmic imaging apparatus (100) according to any one of Claims 8 to 10 when dependent on any one of Claims 5 to 7, wherein
the supporting portion (240) is attached to the base portion (245), and
the pivot (401) is provided on the base portion (245) and aligned with the second axis of rotation (A₂) such that the reflective element (156; 230) mounted on the arm (154; 220) is rotatable about the second axis of rotation (A₂).

12. The ophthalmic imaging apparatus (100) according to Claims 4, 7 and 10, wherein the first threaded portion, the second threaded portion, and the third threaded portion are arranged to extend along a direction aligned to the optical path (P; 211), such that the first screw, the second screw and the third screw can each be rotated about respective axes that are aligned with one another.

13. The ophthalmic imaging apparatus (100) according to any preceding claim, wherein a projection of the arm (154; 220) on a plane perpendicular to the optical path (P; 211) has an area which is less than 10 % of an area of a cross-section of the housing (152; 210) or of the light (L_{C}) collected by the optical system (130) that propagated through the housing (152) during use of the ophthalmic imaging apparatus (100).

14. The ophthalmic imaging apparatus (100) according to any preceding claim, wherein the light source (110) is slidably attached to the ophthalmic imaging apparatus (100) such that a location on the reflective element (156; 230), at which the light beam (L_{B}) generated by the light source (110) is incident on the reflective element (156; 230), is adjustable along an axis perpendicular to the optical path (P; 211).

## Patentansprüche

1. Vorrichtung zur ophthalmologischen Bildgebung (100), umfassend:
eine Lichtquelle (110), angeordnet um einen Lichtstrahl (L_{b}) zu erzeugen;
einen Photodetektor (120);
ein optisches System (130), angeordnet um einen Teil eines Auges (140) mit Licht des Lichtstrahls (L_{b}) zu beleuchten und Licht (L_{C}) von dem beleuchteten Teil des Auges (140) zu sammeln; und
eine optische Baugruppe (150), angeordnet um den Lichtstrahl (L_{b}) von der Lichtquelle (110) zum optischen System (130) zu leiten und das vom optischen System (130) gesammelte Licht (L_{C}) zum Photodetektor (120) weiterzuleiten,
wobei die optische Baugruppe (150) umfasst:
ein Gehäuse (152; 210), durch das sich der Lichtstrahl (L_{b}) von der Lichtquelle (110) zum optischen System (130) in einer ersten Richtung (D₁) entlang eines optischen Pfades (P; 211) während der Verwendung der Vorrichtung zur ophthalmologischen Bildgebung (100) ausbreitet, und durch das sich das vom optischen System (130) gesammelte Licht (L_{C}) zum Photodetektor (120) in einer zweiten Richtung (D₂) entlang des optischen Pfades (P; 211) während der Verwendung der Vorrichtung zur ophthalmologischen Bildgebung (100) ausbreitet, wobei die zweite Richtung (D₂) der ersten Richtung (D₁) entgegengesetzt ist, und das Gehäuse (152; 210) eine Öffnung (153)umfasst, um dem von der Lichtquelle (110) erzeugten Lichtstrahl (L_{b}) Eintritt in das Gehäuse (152; 210) zu ermöglichen;
einen Arm (154; 220), der sich in das Gehäuse (152; 210) hinein erstreckt;
ein an dem Arm (154; 220) montiertes reflektives Element (156; 230), wobei das reflektive Element (156; 230) eine reflektierende Oberfläche (157) zum Reflektieren des durch die Öffnung (153) tretenden Lichtstrahls während der Verwendung der Vorrichtung zur ophthalmologischen Bildgebung (100) umfasst; und
einen justierbaren Befestigungsmechanismus (158), angeordnet um den Arm (154; 220) an dem Gehäuse (152; 210) zu befestigen und eine Justierung des reflektiven Elements (156; 230) durch mindestens eines der folgenden zu ermöglichen:
drehen des reflektiven Elements (156; 230) um eine erste Rotationsachse (A₁), die durch einen Punkt (231) auf der reflektierenden Oberfläche (157) verläuft, wobei die erste Rotationsachse (A₁) senkrecht zum optischen Pfad (P; 211) steht;
drehen des reflektiven Elements (156; 230) um eine zweite Rotationsachse (A₂), die durch den Punkt (231) auf der reflektierenden Oberfläche (157) verläuft, wobei die zweite Rotationsachse (A₂) senkrecht zur ersten Rotationsachse (A₁) und zum optischen Pfad (P; 211) steht; oder
verschieben des reflektiven Elements (156; 230) entlang des optischen Pfades (P; 211),
so dass das reflektive Element (156; 230) während der Verwendung der Vorrichtung zur ophthalmologischen Bildgebung (100) so angeordnet ist, dass es den durch die Öffnung (153) tretenden Lichtstrahl (L_{b}) reflektiert, um diesen entlang des optischen Pfades (P; 211) in der ersten Richtung (D₁) auszubreiten.

2. Vorrichtung zur ophthalmologischen Bildgebung (100) nach Anspruch 1, wobei
der justierbare Befestigungsmechanismus (158) angeordnet ist, eine Justierung des an dem Arm (154; 220) montierten reflektivem Elements (156; 230) durch Drehen des reflektiven Elements (156; 230) um die erste Rotationsachse (A₁) zu ermöglichen, und eine erste dem reflektiven Element (156; 230) zugewandte Kontaktfläche (301) umfasst, wobei die erste Kontaktfläche (301) die Form eines Teils einer Rotationsfläche um die erste Rotationsachse (A₁) aufweist,
der Arm (154; 220) eine oder mehrere zweite Kontaktflächen (302) umfasst, angeordnet um die erste Kontaktfläche (301) an Punkten, die den gleichen Abstand von der ersten Rotationsachse (A₁) haben und jeweils unterschiedliche Winkelpositionen um die erste Rotationsachse (A₁) aufweisen, zu kontaktieren,
und die eine oder die mehreren zweiten Kontaktflächen (302) über die erste Kontaktfläche (301) gleitfähig sind, so dass das reflektive Element (156; 230) um die erste Rotationsachse (A₁) drehbar ist.

3. Vorrichtung zur ophthalmologischen Bildgebung (100) nach Anspruch 2, wobei
der justierbare Befestigungsmechanismus (158) ferner umfasst:
ein erstes bewegliches Element (250), das an dem justierbar Befestigungsmechanismus (158) angebracht ist und ein an einer Seite (304) des Arms (154; 220) anliegendes Ende aufweist, wobei das erste bewegliche Element (250) relativ zum justierbaren Befestigungsmechanismus (158) bewegbar ist um eine Position des Endes des ersten beweglichen Elements (250) relativ zum justierbaren Befestigungsmechanismus (158) zu variieren; und
ein erstes resilientes Element (303), angeordnet um die Seite (304) des Arms (154; 220) gegen das Ende des ersten beweglichen Elements (250) zu drücken, und
das erste bewegliche Element (250) angeordnet ist, bei einer Bewegung relativ zum justierbaren Befestigungsmechanismus (158), zu bewirken, dass sich der Arm (154; 220) so bewegt, dass die eine oder die mehreren zweiten Kontaktflächen (302) über die erste Kontaktfläche (301) gleiten.

4. Vorrichtung zur ophthalmologischen Bildgebung (100) nach Anspruch 3, wobei das an dem justierbaren Befestigungsmechanismus (158) angebrachte erste bewegliche Element (250) eine erste Schraube umfasst, die durch einen ersten Gewindeabschnitt des justierbaren Befestigungsmechanismus (158) geschraubt wurde, wobei ein Ende der ersten Schraube an der Seite (304) des Arms (154; 220) anliegt, und wobei die erste Schraube, wenn sie gedreht wird, um sich durch den ersten Gewindeabschnitt zu bewegen, bewirkt, dass sich der Arm (154; 220) so bewegt, dass die eine oder die mehreren zweiten Kontaktflächen (302) über die erste Kontaktfläche (301) gleiten.

5. Vorrichtung zur ophthalmologischen Bildgebung (100) nach einem der vorhergehenden Ansprüche, wobei der justierbare Befestigungsmechanismus (158) ermöglicht, dass das an dem Arm (154; 220) montierte reflektive Element (156; 230) durch Drehen des reflektiven Elements (156; 230) um die zweite Rotationsachse (A₂) justiert wird, und einen am Arm (154; 220) angebrachten Trägerabschnitt (240) umfasst, der Trägerabschnitt (240) um einen Drehpunkt (401) drehbar ist, welcher mit der zweite Rotationsachse (A₂) ausgerichtet ist, so dass das am Arm (154; 220) montierte reflektive Element (156; 230) um die zweite Rotationsachse (A₂) drehbar ist.

6. Vorrichtung zur ophthalmologischen Bildgebung (100) nach Anspruch 5,
wobei der justierbare Befestigungsmechanismus (158) umfasst:
ein zweites bewegliches Element (251), das an dem justierbaren Befestigungsmechanismus (158) angebracht ist und ein an einer Seite (403) des Trägerabschnitts (240) anliegendes Ende aufweist; und
ein zweites resilientes Element (402), angeordnet um die Seite (403) des Trägerabschnitts (240) gegen das Ende des zweiten beweglichen Elements (251) zu drücken, und
das zweite bewegliche Element (251) angeordnet ist, bei einer Bewegung relativ zum justierbaren Befestigungsmechanismus (158) zu bewirken, dass sich der Trägerabschnitt (240) um den Drehpunkt (401) dreht, wodurch bewirkt wird, dass sich das reflektive Element (156; 230) um die zweite Rotationsachse (A₂) dreht.

7. Vorrichtung zur ophthalmologischen Bildgebung (100) nach Anspruch 6, wobei das an dem justierbaren Befestigungsmechanismus (158) angebrachte zweite bewegliche Element (251) eine zweite Schraube umfasst, die durch einen zweiten Gewindeabschnitt des justierbaren Befestigungsmechanismus (158) geschraubt wurde, wobei ein Ende der zweiten Schraube an der Seite (403) des Trägerabschnitts (240) anliegt, und wobei die zweite Schraube, wenn sie gedreht wird, um sich durch den zweiten Gewindeabschnitt zu bewegen, bewirkt, dass sich der Trägerabschnitt (240) um den Drehpunkt (401) dreht.

8. Vorrichtung zur ophthalmologischen Bildgebung (100) nach einem der vorhergehenden Ansprüche, wobei der justierbare Befestigungsmechanismus (158) es ermöglicht, das an dem Arm (154; 220) montierte reflektive Element (156; 230) durch Verschieben des reflektiven Elements (156; 230) entlang des optischen Pfades (P; 211) zu justieren, der justierbare Befestigungsmechanismus (158) einen Basisabschnitt (245) umfasst, welcher gleitfähig an dem Gehäuse (152; 210) angebracht ist, so dass er entlang des Gehäuses (152; 210) in einer Richtung parallel zum optischen Pfad (P; 211) gleitfähig ist, so dass das an dem Arm (154; 220) montierte reflexionsfähige Element (156; 230) entlang des optischen Pfades (P; 211) verschiebbar ist.

9. Vorrichtung zur ophthalmologischen Bildgebung (100) nach Anspruch 8, wobei die optische Baugruppe ferner umfasst:
ein drittes bewegliches Element (252), das an dem Gehäuse (152; 210) angebracht ist und ein an einer Seite (502) des Basisabschnitts (245) anliegendes Ende aufweist; und
ein drittes resilientes Element (501), angeordnet um, die Seite (502) des Basisabschnitts (245) gegen das Ende des dritten beweglichen Elements (252) zu drücken,
wobei das dritte bewegliche Element (252) angeordnet ist, bei einer Bewegung relativ zum Gehäuse (152; 210), zu bewirken, dass der Basisabschnitt (245) entlang des Gehäuses (152; 210) in der Richtung parallel zum optischen Pfad (P; 211) gleitet, wodurch bewirkt wird, dass das an dem Arm (154; 220) montierte reflektive Element (156; 230) entlang des optischen Pfades (P; 211) verschoben wird.

10. Vorrichtung zur ophthalmologischen Bildgebung (100) nach Anspruch 9, wobei das an dem Gehäuse (152; 210) angebrachte dritte bewegliche Element (252) eine dritte Schraube umfasst, die durch einen dritten Gewindeabschnitt des justierbaren Befestigungsmechanismus (158) geschraubt wurde, wobei ein Ende der dritten Schraube an der Seite (502) des Basisabschnitts (245) anliegt, und wobei die dritte Schraube, wenn sie gedreht wird, um sich durch den dritten Gewindeabschnitt zu bewegen, bewirkt, dass der Basisabschnitt (245) entlang des Gehäuses (152; 210) in der Richtung parallel zum optischen Pfad (P; 211) gleitet.

11. Vorrichtung zur ophthalmologischen Bildgebung (100) nach einem der Ansprüche 8 bis 10, wenn diese von einem der Ansprüche 5 bis 7 abhängen, wobei:
der Trägerabschnitt (240) an dem Basisabschnitt (245) angebracht ist, und
der Drehpunkt (401) an dem Basisabschnitt (245) vorgesehen und mit der zweiten Rotationsachse (A₂) ausgerichtet ist, so dass das an dem Arm (154; 220) montierte reflektive Element (156; 230) um die zweite Rotationsachse (A₂) drehbar ist.

12. Vorrichtung zur ophthalmologischen Bildgebung (100) nach den Ansprüchen 4, 7 und 10, wobei der erste Gewindeabschnitt, der zweite Gewindeabschnitt und der dritte Gewindeabschnitt so angeordnet sind, dass sie sich entlang einer zum optischen Pfad (P; 211) ausgerichteten Richtung erstrecken, so dass die erste Schraube, die zweite Schraube und die dritte Schraube jeweils um entsprechende Achsen gedreht werden können, die zueinander ausgerichtet sind.

13. Vorrichtung zur ophthalmologischen Bildgebung (100) nach einem der vorhergehenden Ansprüche, wobei eine Projektion des Arms (154; 220) auf eine Ebene senkrecht zum optischen Pfad (P; 211) eine Fläche aufweist, die weniger als 10 % einer Querschnittsfläche des Gehäuses (152; 210) oder des vom optischen System (130) gesammelten Lichts (L_{c}) ausmacht, das sich während der Verwendung der Vorrichtung zur ophthalmologischen Bildgebung (100) durch das Gehäuse (152) ausgebreitet hat.

14. Vorrichtung zur ophthalmologischen Bildgebung (100) nach einem der vorhergehenden Ansprüche, wobei die Lichtquelle (110) gleitfähig an der Vorrichtung zur ophthalmologischen Bildgebung (100) angebracht ist, so dass eine Stelle auf dem reflektiven Element (156; 230), an welcher der von der Lichtquelle (110) erzeugte Lichtstrahl (L_{b}) auf das reflektive Element (156; 230) einfällt, entlang einer Achse senkrecht zum optischen Pfad (P; 211) justierbar ist.

## Revendications

1. Appareil d'imagerie ophtalmique (100), comprenant :
une source lumineuse (110) agencée pour générer un faisceau lumineux (L_{B}) ;
un photodétecteur (120) ;
un système optique (130) agencé pour éclairer une partie d'un œil (140) avec de la lumière provenant du faisceau lumineux (L_{B}) et collecter de la lumière (L_{C}) provenant de la partie éclairée de l'œil (140), le système optique 130 comprenant un système de balayage agencé pour effectuer un balayage d'une lumière (L_{I}) provenant de la source lumineuse (110) sur la partie de l'œil (140) et pour collecter la lumière (L_{C}) provenant de la partie de l'œil (140) pendant le balayage, le balayage étant un balayage ponctuel bidimensionnel ou un balayage linéaire ; et
un ensemble optique (150) agencé pour guider le faisceau lumineux (L_{B}) provenant de la source lumineuse (110) vers le système optique (130), et pour acheminer la lumière (L_{C}) collectée par le système optique (130) vers le photodétecteur (120),
l'ensemble optique (150) comprenant :
un boîtier (152 ; 210) à travers lequel le faisceau lumineux (L_{B}) provenant de la source lumineuse (110) se propage vers le système optique (130) dans une première direction (D1) le long d'un chemin optique (P ; 211) pendant une utilisation de l'appareil d'imagerie ophtalmique (100), et à travers lequel la lumière (L_{C}) collectée par le système optique (130) se propage vers le photodétecteur (120) dans une deuxième direction (D2) le long du chemin optique (P ; 211) pendant une utilisation de l'appareil d'imagerie ophtalmique (100), dans lequel la deuxième direction (D2) est opposée à la première direction (D1), et le boîtier (152 ; 210) comprend une ouverture (153) pour permettre au faisceau lumineux (L_{B}) généré par la source lumineuse (110) d'entrer dans le boîtier (152 ; 210) ;
un bras (154 ; 220) qui s'étend dans le boîtier (152 ; 210) ;
un élément réfléchissant (156 ; 230) monté sur le bras (154 ; 220), l'élément réfléchissant (156 ; 230) comprenant une surface réfléchissante (157) pour réfléchir le faisceau lumineux passant à travers l'ouverture (153) pendant une utilisation de l'appareil d'imagerie ophtalmique (100) ; et
un mécanisme de fixation réglable (158) agencé pour fixer le bras (154 ; 220) au boîtier (152 ; 210) et pour permettre à l'élément réfléchissant (156 ; 230) d'être réglé par au moins l'une des opérations suivantes :
la rotation de l'élément réfléchissant (156 ; 230) autour d'un premier axe de rotation (A₁) passant par un point (231) sur la surface réfléchissante (157), le premier axe de rotation (A₁) étant perpendiculaire au chemin optique (P ; 211) ;
la rotation de l'élément réfléchissant (156 ; 230) autour d'un deuxième axe de rotation (A₂) passant par le point (231) sur la surface réfléchissante (157), le deuxième axe de rotation (A₂) étant perpendiculaire au premier axe de rotation (A₁) et au chemin optique (P ; 211) ; ou
la translation de l'élément réfléchissant (156 ; 230) le long du chemin optique (P ; 211),
de telle sorte que, pendant une utilisation de l'appareil d'imagerie ophtalmique (100), l'élément réfléchissant (156 ; 230) soit agencé pour réfléchir le faisceau lumineux (L_{B}) passant à travers l'ouverture (153) afin de se propager le long du chemin optique (P ; 211) dans la première direction (D1).

2. Appareil d'imagerie ophtalmique (100) selon la revendication 1, dans lequel
le mécanisme de fixation réglable (158) est agencé pour permettre à l'élément réfléchissant (156 ; 230) monté sur le bras (154 ; 220) d'être réglé en faisant tourner l'élément réfléchissant (156 ; 230) autour du premier axe de rotation (A₁), et comprend une première surface de contact (301) qui fait face à l'élément réfléchissant (156 ; 230), dans lequel la première surface de contact (301) a la forme d'une partie d'une surface de révolution autour du premier axe de rotation (A₁),
le bras (154 ; 220) comprend une ou plusieurs deuxièmes surfaces de contact (302) agencées pour entrer en contact avec la première surface de contact (301) en des points qui sont équidistants du premier axe de rotation (A₁) et ont des positions angulaires respectives différentes autour du premier axe de rotation (A₁), et
la ou les deuxièmes surfaces de contact (302) peuvent coulisser sur la première surface de contact (301) de telle sorte que l'élément réfléchissant (156 ; 230) peut tourner autour du premier axe de rotation (A₁).

3. Appareil d'imagerie ophtalmique (100) selon la revendication 2, dans lequel
le mécanisme de fixation réglable (158) comprend en outre :
un premier élément mobile (250) fixé au mécanisme de fixation réglable (158) et ayant une extrémité en butée contre un côté (304) du bras (154 ; 220), dans lequel le premier élément mobile (250) est mobile par rapport au mécanisme de fixation réglable (158) afin de faire varier une position de l'extrémité du premier élément mobile (250) par rapport au mécanisme de fixation réglable (158) ; et
un premier élément résilient (303) agencé pour forcer le côté (304) du bras (154 ; 220) contre l'extrémité du premier élément mobile (250), et
le premier élément mobile (250), lorsqu'il est déplacé par rapport au mécanisme de fixation réglable (158), est agencé pour amener le bras (154 ; 220) à se déplacer de telle sorte que la ou les deuxièmes surfaces de contact (302) glissent sur la première surface de contact (301).

4. Appareil d'imagerie ophtalmique (100) selon la revendication 3, dans lequel le premier élément mobile (250) fixé au mécanisme de fixation réglable (158) comprend une première vis qui a été vissée à travers une première partie filetée du mécanisme de fixation réglable (158), dans lequel une extrémité de la première vis bute contre le côté (304) du bras (154 ; 220), et dans lequel la première vis, lorsqu'elle est tournée pour se déplacer à travers la première partie filetée, provoque le déplacement du bras (154 ; 220) de telle sorte que la ou les deuxièmes surfaces de contact (302) glissent sur la première surface de contact (301).

5. Appareil d'imagerie ophtalmique (100) selon l'une quelconque des revendications précédentes, dans lequel le mécanisme de fixation réglable (158) permet d'ajuster l'élément réfléchissant (156 ; 230) monté sur le bras (154 ; 220) en tournant l'élément réfléchissant (156 ; 230) autour du deuxième axe de rotation (A₂), et comprend une partie de support (240) fixée au bras (154 ; 220), la partie de support (240) pouvant tourner autour d'un pivot (401) qui est aligné avec le deuxième axe de rotation (A₂) de telle sorte que l'élément réfléchissant (156 ; 230) monté sur le bras (154 ; 220) puisse tourner autour du deuxième axe de rotation (A₂).

6. Appareil d'imagerie ophtalmique (100) selon la revendication 5, dans lequel
le mécanisme de fixation réglable (158) comprend :
un deuxième élément mobile (251) fixé au mécanisme de fixation réglable (158) et ayant une extrémité en butée contre un côté (403) de la partie de support (240) ; et
un deuxième élément résilient (402) agencé pour forcer le côté (403) de la partie de support (240) contre l'extrémité du deuxième élément mobile (251), et
le deuxième élément mobile (251), lorsque déplacé par rapport au mécanisme de fixation réglable (158), est agencé pour amener la partie de support (240) à tourner autour du pivot (401), amenant ainsi l'élément réfléchissant (156 ; 230) à tourner autour du deuxième axe de rotation (A₂).

7. Appareil d'imagerie ophtalmique (100) selon la revendication 6, dans lequel le deuxième élément mobile (251) fixé au mécanisme de fixation réglable (158) comprend une deuxième vis qui a été vissée à travers une deuxième partie filetée du mécanisme de fixation réglable (158), dans lequel une extrémité de la deuxième vis bute contre le côté (403) de la partie de support (240), et dans lequel la deuxième vis, lorsque tournée pour se déplacer à travers la deuxième partie filetée, provoque la rotation de la partie de support (240) autour du pivot (401).

8. Appareil d'imagerie ophtalmique (100) selon l'une quelconque des revendications précédentes, dans lequel le mécanisme de fixation réglable (158) permet d'ajuster l'élément réfléchissant (156 ; 230) monté sur le bras (154 ; 220) en translatant l'élément réfléchissant (156 ; 230) le long du chemin optique (P ; 211), le mécanisme de fixation réglable (158) comprenant une partie de base (245) qui est fixée de manière coulissante au boîtier (152 ; 210) de manière à pouvoir coulisser le long du boîtier (152 ; 210), dans une direction parallèle au chemin optique (P ; 211), de telle sorte que l'élément réfléchissant (156 ; 230) monté sur le bras (154 ; 220) puisse être translaté le long du chemin optique (P ; 211).

9. Appareil d'imagerie ophtalmique (100) selon la revendication 8, dans lequel l'ensemble optique comprend en outre :
un troisième élément mobile (252) fixé au boîtier (152 ; 210) et ayant une extrémité en butée contre un côté (502) de la partie de base (245) ; et
un troisième élément résilient (501) agencé pour forcer le côté (502) de la partie de base (245) contre l'extrémité du troisième élément mobile (252),
dans lequel le troisième élément mobile (252), lorsque déplacé par rapport au boîtier (152 ; 210), est agencé pour amener la partie de base (245) à coulisser le long du boîtier (152 ; 210), dans la direction parallèle au chemin optique (P ; 211), provoquant ainsi la translation de l'élément réfléchissant (156 ; 230) monté sur le bras (154 ; 220) le long du chemin optique (P ; 211).

10. Appareil d'imagerie ophtalmique (100) selon la revendication 9, dans lequel le troisième élément mobile (252) fixé au boîtier (152 ; 210) comprend une troisième vis qui a été vissée à travers une troisième partie filetée du mécanisme de fixation réglable (158), dans lequel une extrémité de la troisième vis bute contre le côté (502) de la partie de base (245), et dans lequel la troisième vis, lorsque tournée pour se déplacer à travers la troisième partie filetée, amène la partie de base (245) à coulisser le long du boîtier (152 ; 210), dans la direction parallèle au chemin optique (P ; 211).

11. Appareil d'imagerie ophtalmique (100) selon l'une quelconque des revendications 8 à 10 lorsque dépendant de l'une quelconque des revendications 5 à 7, dans lequel
la partie de support (240) est fixée à la partie de base (245), et
le pivot (401) est prévu sur la partie de base (245) et aligné avec le deuxième axe de rotation (A₂) de telle sorte que l'élément réfléchissant (156 ; 230) monté sur le bras (154 ; 220) puisse tourner autour du deuxième axe de rotation (A₂).

12. Appareil d'imagerie ophtalmique (100) selon les revendications 4, 7 et 10, dans lequel la première partie filetée, la deuxième partie filetée et la troisième partie filetée sont agencées de manière à s'étendre le long d'une direction alignée avec le chemin optique (P ; 211), de telle sorte que la première vis, la deuxième vis et la troisième vis peuvent chacune tourner autour d'axes respectifs qui sont alignés les uns avec les autres.

13. Appareil d'imagerie ophtalmique (100) selon l'une quelconque des revendications précédentes, dans lequel une projection du bras (154 ; 220) sur un plan perpendiculaire au chemin optique (P ; 211) a une aire qui est inférieure à 10 % d'une aire d'une section transversale du boîtier (152 ; 210) ou de la lumière (L_{C}) collectée par le système optique (130) qui s'est propagée à travers le boîtier (152) pendant une utilisation de l'appareil d'imagerie ophtalmique (100).

14. Appareil d'imagerie ophtalmique (100) selon l'une quelconque des revendications précédentes, dans lequel la source lumineuse (110) est fixée de manière coulissante à l'appareil d'imagerie ophtalmique (100) de telle sorte qu'un emplacement sur l'élément réfléchissant (156 ; 230), au niveau duquel le faisceau lumineux (L_{B}) généré par la source lumineuse (110) est incident sur l'élément réfléchissant (156 ; 230), est réglable le long d'un axe perpendiculaire au chemin optique (P ; 211).
